(19) Europäisches Patentamt
European Patent Office
Office européen des brevets

(11) **EP 2 023 972 B1**

(12) **EUROPÄISCHE PATENTSCHRIFT**

(45) Veröffentlichungstag und Bekanntmachung des Hinweises auf die Patenterteilung:
**01.04.2015 Patentblatt 2015/14**

(21) Anmeldenummer: **07764573.7**

(22) Anmeldetag: **06.06.2007**

(51) Int Cl.:
**A61M 1/16** (2006.01)  **A61M 1/34** (2006.01)

(86) Internationale Anmeldenummer:
**PCT/EP2007/004993**

(87) Internationale Veröffentlichungsnummer:
**WO 2007/140993 (13.12.2007 Gazette 2007/50)**

(54) **VORRICHTUNG UND VERFAHREN ZUR STEUERUNG EINER EXTRAKORPORALEN BLUTBEHANDLUNGSVORRICHTUNG**

DEVICE AND METHOD FOR CONTROLLING AN EXTRACORPOREAL BLOOD TREATMENT DEVICE

DISPOSITIF ET PROCÉDÉ DE COMMANDE D'UN SYSTÈME DE TRAITEMENT SANGUIN EXTRACORPOREL

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **08.06.2006 DE 102006026999**
**17.08.2006 DE 102006038545**

(43) Veröffentlichungstag der Anmeldung:
**18.02.2009 Patentblatt 2009/08**

(73) Patentinhaber: **Fresenius Medical Care Deutschland GmbH**
**61352 Bad Homburg v.d.H. (DE)**

(72) Erfinder:
• **HILGERS, Peter**
**97453 Schonungen (DE)**
• **JONAS, Jörg**
**61273 Wehrheim (DE)**

(74) Vertreter: **Oppermann, Frank et al**
**OANDO Oppermann & Oppermann LLP**
**Washingtonstrasse 75**
**65189 Wiesbaden (DE)**

(56) Entgegenhaltungen:
**US-A- 5 092 836        US-A1- 2003 230 533**
**US-A1- 2005 251 086**

EP 2 023 972 B1

**Beschreibung**

Die Erfindung bezieht sich auf eine Vorrichtung zur Steuerung einer extrakorporalen Blutbehandlungsvorrichtung, insbesondere eine Hämodialysevorrichtung oder Hämofiltrationsvorrichtung oder Hämodiafiltrationsvorrichtung, die einen Dialysator oder Filter aufweist, der durch eine semipermeable Membran in eine erste und zweite Kammer unterteilt ist. Darüber hinaus betrifft die Erfindung eine extrakorporale Blutbehandlungsvorrichtung mit einer derartigen Steuerungsvorrichtung sowie ein Verfahren zur Steuerung einer extrakorporalen Blutbehandlungsvorrichtung.

Als eine extrakorporale Blutbehandlung ist die Hämodialyse bekannt, bei der das zu behandelnde Blut in einem extrakorporalen Blutkreislauf die Blutkammer eines durch eine semipermeable Membran in die Blutkammer und eine Dialysierflüssigkeitskammer unterteilen Dialysators mit einer bestimmten Blutflussrate durchströmt und Dialysierflüssigkeit die Dialysierflüssigkeitskammer des Dialysators mit einer bestimmten Dialysierflüssigkeitsrate durchströmt. Neben der Hämodialyse ist die Hämofiltration als Blutbehandlung bekannt. Die Hämodiafiltration schließt sowohl die Hämodialyse als auch die Hämofiltration ein.

Der Stoffaustausch im Dialysator hat sowohl konvektiven als auch diffusiven Charakter. Beim diffusiven Stoffaustausch ist für die betreffende Substanz der Massentransfer pro Zeiteinheit über die Membran proportional dem Konzentrationsgradienten zwischen Blut und Dialysierflüssigkeit; beim konvektiven Stofftransport hängt der Massentransfer von der Filtratmenge ab, da die Konzentration filtrierbarer Substanzen sowohl im Blut als auch im Filtrat gleich ist (Blutreinigungsverfahren, Georg-Thieme-Verlag Stuttgart, New York, 4. Aufl., 1990, Seiten 11 bis 13).

Da sich das Konzentrationsgefälle während der Dialysebehandlung ständig verringert, kann für die pro Zeiteinheit ausgetauschte Substanzmenge kein fester Zahlenwert angegeben werden. Eine konzentrationsunabhängige Messgröße für die Leistungsfähigkeit eines Dialysators stellt die Clearance dar.

Die Clearance einer Substanz ist der Teilstrom des Gesamtstroms durch den Dialysator, der von der betreffenden Substanz vollständig befreit ist. Die Dialysance ist ein weiterer Begriff zur Bestimmung der Leistungsfähigkeit eines Dialysators, bei der auch Substanzen, die in der Dialysierflüssigkeit enthalten sind, Berücksichtigung finden.

Für die Bestimmung der Dialysance D bzw. Clearance K für eine bestimmte Substanz, beispielsweise Natrium, ergibt sich bei einer Ultrafiltration gleich 0 das Folgende.

Die Dialysance D ist gleich dem Verhältnis zwischen dem blutseitigen Massentransport für die betreffende Substanz Qb(cbi-cbo) und der Konzentrationsdifferenz der Substanz zwischen dem Blut und der Dialysierflüssigkeit am jeweiligen Eingang des Dialysators (cbi-cdi).

$$D = Qb \frac{(cbi - cbo)}{cbi - cdi} \qquad (1)$$

Aus Gründen der Massenbilanz gilt

$$Qb \cdot (cbi - cbo) = -Qd \cdot (cdi - cdo) \qquad (2)$$

aus (1) und (2) folgt für die Dialysance dialysatseitig:

$$D = -Qd \frac{(cdi - cdo)}{cbi - cdi} \qquad (3)$$

Dabei sind in (1) bis (3):

Qb= effektiver Blutfluss
Qd= Dialysierflüssigkeitsfluss
Cb: Konzentration der Substanz im Lösungsvolumen des Blutes
Cd= Konzentration der Substanz in der Dialysierflüssigkeit
i= Eingang des Dialysators
o= Ausgang des Dialysators.

Die EP 0 428 927 A1 beschreibt ein Verfahren zur in-vivo-Bestimmung von Parametern der Hämodialyse, bei dem der Dialysat-Elektrolyttransfer jeweils bei zwei unterschiedlichen Dialysat-Eingangskonzentrationen gemessen wird. Unter der Annahme, dass die Bluteingangskonzentration konstant ist, wird nach dem bekannten Verfahren die Dialysance dadurch bestimmt, dass die Differenz zwischen den Differenzen der Dialysierflüssigkeits-Ionenkonzentration an der Eingangsseite und der Ausgangsseite des Dialysators zum Zeitpunkt der ersten und zweiten Messung bestimmt wird, diese durch die Differenz der Dialysierflüssigkeits-Ionenkonzentration an der Eingangsseite zum Zeitpunkt der ersten Messung und der zweiten Messung geteilt wird und mit dem Dialysierflüssigkeitsfluss multipliziert wird.

Ein weiteres Verfahren zur Bestimmung der Dialysance beschreibt die US 6,702,774 B1. Bei dem bekannten Verfahren wird stromauf des Dialysators als Bolus eine bestimmte Menge einer Substanz zugegeben, deren Dialysance bestimmt werden soll, und die Dialysance wird aus der zugegebenen Stoffmenge stromauf des Dialysators, dem Integral der Stoffkonzentration über die Zeit stromab des Dialysators sowie dem Dialysierflüssigkeitsfluss berechnet.

Des weiteren ist ein Verfahren zur Bestimmung der maximalen Dialysance eines Dialysators aus der DE 197 39 100 C1 bekannt.

Den Zusammenhang zwischen Clearance und Dialysance einerseits und Blut- und Dialysierflüssigkeitsfluss andererseits haben Sigdell und Tersteegen für die Dialyse ohne Ultrafiltration untersucht (Sigdell J., Tersteegen, B.: Clearance of a Dialyzer under varying Operation Conditions; Artificial Organs 10(3): 219-225,1986). Sigdell und Tersteegen haben festgestellt, dass es in der Praxis zur Vergrößerung der Clearance oder Dialysance nicht sinnvoll erscheint, einen Dialysierflüssigkeitsfluss einzustellen, der größer als der doppelte Blutfluss ist. Es sind verschiedene Methoden vorgeschlagen worden, wie der Einfluss der Ultrafiltration auf die Clearance berücksichtigt werden kann. Jedoch ist der Einfluss der Ultrafiltration bei den typischen Flüssen (Qf=15 ml/min, Qd=500 ml/min, Qb=300 ml/min) relativ gering und kann vernachlässigt werden. Werynski und Waniewski haben den Zusammenhang zwischen Flüssen und resultierender Clearance verallgemeinert und die Hämdiafiltration behandelt. Die Hämodialyse ist darin als Spezialfall enthalten (Werynski A. und Waniewski, J.: Theoretical Description of Mass Transport in Medical Membrane Devices, Artificial Organs 19, S. 420-427 (1995)).

Die bekannten Dialysevorrichtungen werden mit einem Blutfluss betrieben, der vom behandelnden Arzt innerhalb vorgegebener Grenzen eingestellt wird, wobei der Dialysierflüssigkeitsfluss ebenfalls innerhalb vorgegebener Grenzen, die im Allgemeinen zwischen 500 ml/min und 800 ml/min liegen, eingestellt wird. Daraus ergibt sich die Dialysedosis, die sich aus dem Quotienten (K T/V) von dem Produkt der Clearance K mit der effektiven Behandlungszeit T und dem Verteilungsvolumen V berechnet.

In der Praxis wird heute gefordert, dass der Quotient (K T/V) für Harnstoff größer als ein vorgegebener Grenzwert, insbesondere größer als 1,3 ist. Dabei ist das Verteilungsvolumen V von dem Patienten abhängig, so dass der Arzt bei der Blutbehandlung nur die vom Blut- und Dialysierflüssigkeitsfluss abhängige Clearance K und die Behandlungszeit T vorgeben kann. Folglich ergibt sich bei einer gewünschten Behandlungszeit rechnerisch ein bestimmter Wert für die Clearance oder Dialysance, die während einer Behandlung sichergestellt werden sollte, um die geforderte Dialysedosis zu erreichen. Wenn sich aber die Blutflussrate oder Dialysierflüssigkeitsrate während der Behandlung ändert, kann eine bestimmte Clearance nicht gewährleistet werden.

Die US 5,092,836 beschreibt ein Hämodialyseverfahren, das eine Ersparnis von Dialysierflüssigkeit erlauben soll. Das Verfahren sieht davon ab, einen festen Wert für den Blutfluss und den Dialysierflüssigkeitsfluss vorzugeben. Vielmehr soll ein Dialysierflüssigkeitsfluss vorgegeben werden, der in einem konstanten Verhältnis zu dem vorgegebenen Blutfluss steht.

Des weiteren ist aus der WO 2004/022135 A1 eine Dialysevorrichtung bekannt, bei der die Dialysance gemessen und durch Veränderung der Ultrafiltrationsrate sichergestellt wird, dass sowohl die Dialysedosis KT/V als auch der gewünschte Gewichtsverlust des Patienten zum gleichen Zeitpunkt erreicht wird.

Die US-A-5,744,031 beschreibt ein Verfahren zur Steuerung einer Blutbehandlung, bei dem zur Bestimmung der Dialysance eine Leitfähigkeitsmessung durchgeführt wird, wobei der gemessene Wert für die Dialysance mit einem gewünschten Wert verglichen wird, um die Blutflussrate oder Dialysierflüssigkeitsrate derart zu ändern, dass der Ist-Wert für die Dialysance dem Soll-Wert entspricht. Das bekannte Verfahren ist insofern nachteilig, als eine Leitfähigkeitsmessung zur Bestimmung der Dialysance während der Blutbehandlung erforderlich ist. Eine fortlaufende Leitfähigkeitsmessung ist aber nicht nur mit einem erhöhten Aufwand verbunden, sondern setzt auch einer schnellen Regelung Grenzen, da für die einzelnen Messungen relativ viel Zeit erforderlich ist, um die Messgrößen mit der erforderlichen Genauigkeit erfassen zu können.

Aus der US 2003/0230533 A1 ist sowohl für die Hämodialyse als auch Hämofiltration und der Kombination beider Verfahren, d.h. die Hämodiafiltration, der Zusammenhang zwischen den Flussraten einerseits und der Clearance oder Dialysance andererseits bekannt. Auf diese Druckschrift wird im Folgenden zum Zwecke der Offenbarung ausdrücklich Bezug genommen.

Der Erfindung liegt die Aufgabe zu Grunde, eine Vorrichtung zur Steuerung einer extrakorporalen Blutbehandlungsvorrichtung zu schaffen, die eine optimierte Blutbehandlung mit einer vorgegebenen Clearance oder Dialysance erlaubt. Eine weitere Aufgabe der Erfindung liegt darin, eine Blutbehandlungsvorrichtung mit einer derartigen Steuerungsvorrichtung zu schaffen. Eine Aufgabe der Erfindung ist auch, ein Verfahren zum Steuern einer extrakorporalen Blutbehandlungsvorrichtung anzugeben, das eine optimierte Blutbehandlung mit einer vorgegebenen Clearance oder Dialysance ermöglicht. Schließlich ist Aufgabe der Erfindung ein Computerprogrammprodukt für eine derartige Steuerungsvorrichtung bereitzustellen.

Die Lösung dieser Aufgaben erfolgt erfindungsgemäß mit den Merkmalen der Patentansprüche 1, 4, 13, 14, 15, 18 und 27. Vorteilhafte Ausführungsformen der Erfindung sind Gegenstand der Unteransprüche.

Die erfindungsgemäße Steuerungsvorrichtung bzw. das erfindungsgemäße Verfahren ist für eine extrakorporale Blutbehandlungsvorrichtung bestimmt, die sowohl als Hämodialysevorrichtung als auch als Hämofiltrationsvorrichtung ausgebildet sein kann. Darüber hinaus kann die erfindungsgemäße Steuerungsvorrichtung und das Verfahren für eine Hämodiafiltrationsvorrichtung bestimmt sein.

Die unterschiedlichen Anwendungsfälle unterscheiden sich dadurch, dass bei den einzelnen Behandlungsverfahren unterschiedliche Flussraten eine Rolle spielen, die jeweils Einfluss auf die Dialysance oder Clearance haben. So sieht die Hämodiafiltration neben der Blutflussrate und Dialysierflüssigkeitsrate auch die Möglichkeit der Änderung der Ultrafiltrationsrate oder der Substituatrate vor. Da für alle Anwendungsfälle aber die Abhängigkeit der Dialysance oder Clearance von den einzelnen Flussarten bekannt ist, unterscheiden sich die alternativen Ausführungsformen der erfindungsgemäßen Steuerungvorrichtung nicht grundsätzlich voneinander.

Bei dem allgemeinen Fall der extrakorporalen Blutbehandlung, der alle Behandlungsverfahren einschließt, wird von einer Austauscheinheit gesprochen, die entweder als Dialysator oder als Filter für die Spezialfälle der Hämodialyse bzw. Hämofiltration ausgebildet sein kann.

Die extrakorporale Hämodialysevorrichtung beispielsweise, die eine Ausführungsform betrifft, verfügt über einen Dialysator, der durch eine semipermiable Membran in eine Blutkammer und eine Dialysierflüssigkeitskammer unterteilt ist, und eine Blutpumpe zum Fördern von Blut durch die Blutkammer mit einer bestimmten Blutflussrate $Q_b$ und eine Dialysierflüssigkeitspumpe zum Fördern von Dialysierflüssigkeit durch die Dialysierflüssigkeitskammer mit einer bestimmten Dialysierflüssigkeitsrate $Q_d$.

Die erfindungsgemäße Steuerungsvorrichtung kann eine selbständige Baugruppe bilden oder Bestandteil der extrakorporalen Blutbehandlungsvorrichtung sein. Da wesentliche Bauteile der erfindungsgemäßen Steuerungsvorrichtung, beispielsweise eine Steuereinheit (Microprozessor) und eine Speichereinheit ohnehin Bestandteil der bekannten Blutbehandlungsvorrichtungen sind, kann die erfindungsgemäße Steuerungsvorrichtung ohne größeren technischen Aufwand bei den bekannten Blutbehandlungsvorrichtungen vorgesehen werden. Wenn die erforderliche Hardware vollständig zur Verfügung steht, kann die Bereitstellung des erfindungsgemäßen Computerprogrammprodukts genügen.

Die erfindungsgemäße Vorrichtung und das erfindungsgemäße Verfahren gehen davon aus, dass vor der Behandlung unterschiedliche Flüsse öder Flussraten vorgegeben und/oder während der Blutbehandlung unterschiedliche Flüsse bzw. Flussraten, beispielsweise Blutflussraten oder Dialysierflüssigkeitsraten geändert werden.

Bei einer Vorgabe oder Änderung einer Blutflussrate beispielsweise wird vor bzw. während der Blutbehandlung die Dialysierflüssigkeitsrate derart vorgegeben oder verändert, dass vorzugsweise bei einer vorgegebenen Behandlungsdauer eine gewünschte Clearance oder Dialysance beibehalten wird. Grundsätzlich kann die Dialysierflüssigkeitsrate nur vorgegeben, muss aber nicht automatisch eingestellt werden. Vorzugsweise wird die Dialysierflüssigkeitsrate aber auch maschinenseitig eingestellt, bei der die gewünschte Clearance oder Dialysance beibehalten wird. Dies kann automatisch geschehen oder nach einer Bestätigung durch einen Benutzer.

Die Blutflussrate kann während der Behandlung einmal oder mehrmals, grundsätzlich auch kontinuierlich verändert werden, wobei die Dialysierflüssigkeitsrate dann immer so eingestellt wird, dass die gewünschte Clearance oder Dialysance vorzugsweise innerhalb der vorgegebenen Behandlungsdauer beibehalten wird. Umgekehrt wird bei einer Veränderung der Dialysierflüssigkeitsrate die Blutflussrate derart eingestellt, dass vorzugsweise innerhalb der vorgegebenen Behandlungsdauer die gewünschte Clearance oder Dialysance beibehalten wird. Entscheidend ist, dass die jeweilige Flussrate nicht auf der Grundlage einer Messung der Clearance oder Dialysance, beispielsweise einer Leitfähigkeitsmessung ermittelt wird, sondern auf der Grundlage der bekannten Abhängigkeit der Clearance oder Dialysance von den Flussarten berechnet wird. Damit ist es möglich, die Flussraten schnell und kontinuierlich anzupassen, um die gewünschte Clearance oder Dialysance während der Behandlung sicherzustellen.

Grundsätzlich kann sowohl die Dialysierflüssigkeitsrate als auch die Blutflussrate verändert werden. In der Praxis wird aber im Allgemeinen eine Veränderung der Blutflussrate auftreten. Dann wird nur die Dialysierflüssigkeitsrate entsprechend angepasst.

Die gewünschte Clearance oder Dialysance wird vorzugsweise vor der Blutbehandlung mit einer Eingabeeinheit eingegeben und in einer Speichereinheit gespeichert. Damit stellt die gewünschte Dialysance oder Clearance einen Soll-Wert für die Steuerung dar.

Eine weitere mögliche Ausführungsform sieht nicht die Eingabe gewünschter Werte für die Clearance oder Dialysance, sondern die Messung dieser Größen vor. Vorzugsweise wird die Clearance oder Dialysance zu Beginn der Dialysebehandlung gemessen, wobei die Steuerung im Laufe der Behandlung dann ohne weitere Messung der Dialysance oder Clearance erfolgt. Damit stellt der Messwert den Soll-Wert dar, der mit der Blutbehandlung erreicht werden soll, auch wenn die Dialysierflüssigkeitsrate oder Blutflussrate während der Behandlung verändert wird. Die Verfahren zum Messen der Dialysance oder Clearance als solche gehören zum Stand der Technik.

Der Zusammenhang zwischen der gewünschten Clearance oder Dialysance einerseits und beispielsweise der Blutflussrate oder Dialysierflüssigkeitsrate andererseits kann durch eine Gleichung beschrieben werden, die neben den Größen der Blutflussrate und der Dialysierflüssigkeitsrate nur einen weiteren Koeffizienten k0A enthält, der im Wesentlichen von der Oberfläche der semipermeablen Membran des Dialysators und dem Diffusionswiderstand der Membran des Dialysators abhängig ist. Dieser Koeffizient k0A kann für verschiedene Typen von Dialysatoren vor Beginn der Blutbehandlung vorgegeben und gespeichert werden. Es ist aber auch möglich, den Koeffizienten k0A dadurch zu bestimmen, dass bei einer vorgegebenen Blutflussrate und Dialysierflüssigkeitsrate die Clearance oder Dialysance gemessen und der Koeffizient mit der Gleichung berechnet wird, die den Zusammenhang zwischen Clearance oder Dialysance einerseits und Blutfluss- und Dialysierflüssigkeitsrate andererseits beschreibt.

Im folgenden wird ein Ausführungsbeispiel der Erfindung unter Bezugnahme auf die Zeichnungen im Einzelnen erläutert. Es zeigen:

Fig. 1        eine vereinfachte schematische Darstellung der Flüssigkeitsverhältnisse bei der Hämodialyse,

Fig. 2a       eine vereinfachte schematische Darstellung der Flüssigkeitsverhältnisse bei der Hämofiltration mit Prädilution,

Fig. 2b       eine vereinfachte schematische Darstellung der Flüssigkeitsverhältnisse bei der Hämofiltration mit Postdilution und

Figur 3       in vereinfachter schematischer Darstellung die wesentlichen Komponenten einer erfindungsgemäßen extrakorporalen Blutbehandlungsvorrichtung zusammen mit einer erfindungsgemäßen Steuerungsvorrichtung für den Fall der Hämodialyse.

Nachfolgend wird der Zusammenhang zwischen der Dialysance oder Clearance und den Flussraten bei der extrakorporalen Blutbehandlung erläutert. Die Abhängigkeit der Clearance und Dialysance von den Flussraten ist in der US 2003/0230533 A1 im Einzelnen beschrieben, auf die zum Zwecke der Offenbarung ausdrücklich Bezug genommen wird.

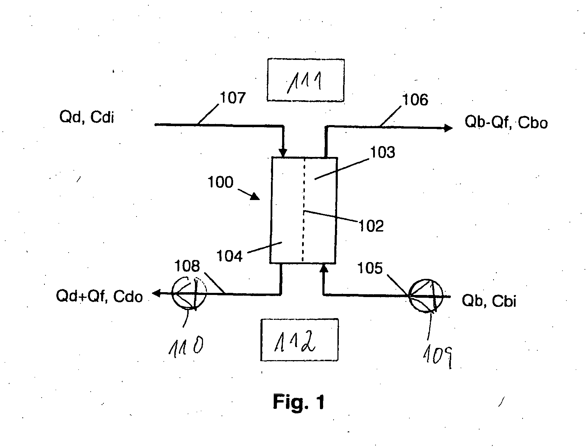

Fig. 1 zeigt den Fall einer Hämodialysebehandlung. Der Hämodialysator 100 wird durch eine semipermeable Membran 102 in zwei Kammern 103 und 104 geteilt, wobei in die erste Kammer 104 frische Dialysierflüssigkeit über eine Dialysierflüssigkeitszuführleitung 107 mit dem Fluss Qd und einer physikalisch-chemischen Eigenschaft Cdi fließt. Aus dieser Kammer 104 fließt über eine Dialysierflüssigkeitsabführleitung 108 ein um den zu entfernenden Ultrafiltrationsfluss Qf vergrößerter Fluss Qd+Qf mit der physikalisch-chemischen Eigenschaft Cdo ab. In die zweite Kammer 103 strömt Blut über eine Blutzuführleitung 105 mit dem Fluss Qb und der physikalisch-chemischen Eigenschaft Cbi. Diese Kammer 103 verläßt ein Blutfluss über die Blutabführleitung 106, der um den Ultrafiltrationsfluss Qf vermindert ist und die physikalisch-chemische Eigenschaft Cbo aufweist. Das Blut wird mit einer Blutpumpe 109 und die Dialysierflüssigkeit mit einer Dialysierflüssigkeitspumpe 110 gefördert, deren Förderraten den Blutfluss bzw. Dialysierflüssigkeitsfluss bestimmen. Der Ultrafiltrationsfluss wird von einer Ultrafiltrationseinrichtung vorgegeben, die mit 111 bezeichnet wird. Die Überwachung der Blutbehandlung und Einstellung der jeweiligen Flussraten über-nimmt eine Steuereinheit 112, die über eine Recheneinheit verfügt.

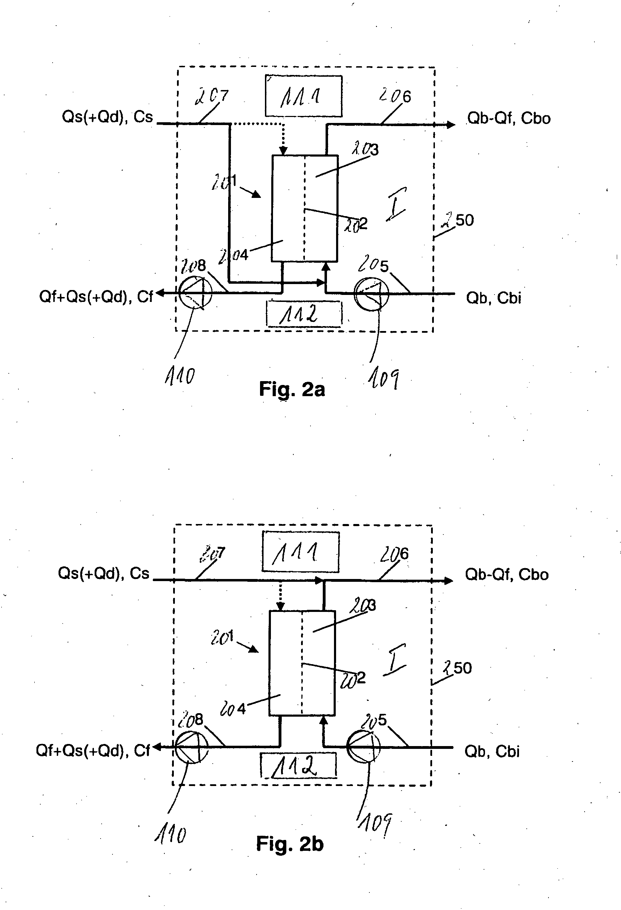

Die Fig. 2a und 2b zeigen eine entsprechend schematisierte Hämofiltrationsvorrichtung, bei der ein durch eine semipermable Membran 202 in zwei Kammern 203 und 204 unterteilter Hämofilter 201 als Austauscheinheit vorgesehen ist. Bezüglich der Blutseite gelten die gleichen Begriffe wie in Fig. 1 erläutert. Weiterhin ist ein Substitutionsflüssigkeitszuführleitung 207 vorgesehen, die direkt entweder mit der Blutzuführleitung 205 (Prädilution, Fig. 2a) oder Blutabführleitung 206 (Postdilution, Fig. 2b) verbunden ist. Durch diese Leitung wird dem extrakorporalen Blutkreislauf I direkt, also nicht über die Membran 202, Substitutionsflüssigkeit mit dem Fluss Qs und der physikalisch-chemischen Eigenschaft Cs zugegeben. Des weiteren wird über die Membran 202 dem Blut Flüssigkeit mit dem Fluss Qo=Qf+Qs entzogen, die in die erste Kammer 204 einströmt und diese Kammer über die Ultrafiltratabführleitung 208 mit der physikalisch-chemischen Eigenschaft Cf verlässt. Die Ultrafiltrationseinrichtung und die Steuereinheit sind wieder mit den Bezugziffern 111 und 112 versehen.

In den Fig. 2a und 2b ist des weiteren ein gepunkteter Verlauf angegeben, der von der Substitutionszuführleitung 207 abzweigt und zur ersten Kammer 204 führt. Dieser Weg wird bei einer Hämodiafiltrationsanwendung zusätzlich durchflossen. Die Flussverhältnisse ändern sich dann insofern, als die in Klammern angegebenen Terme für den Dialysierflüssigkeitfluss Qd hinzutreten. Der die Ultrafiltratabführleitung durchfließende Fluss beläuft sich dann auf Qo=Qf+Qs+Qd. Für die physikalisch-chemischen Eigenschaften Cs und Cf werden die gleichen Bezeichnungen weiterverwendet. Für den in den Fig. 2a und 2b gezeigten Verlauf bleibt Cs durch die Hämodiafiltration unverändert. Der Wert für Cf wird sich jedoch ändern, da nun der Teil Qd des Flusses Qs+Qd mit der physikalisch-chemischen Eigenschaft Cs die erste Kammer durchfließt und sich mit dem durch die Membran hinzutretenden Fluss Qs+Qf vermischt, um gemeinsam über die Ultrafiltratabführleitung 208 abgeführt zu werden.

Nachfolgend wird der Zusammenhang zwischen den Flussraten und der Dialysance beschrieben. Es wird angenommen, dass eine bestimmte Dialysance und mindestens eine der relevanten Flussraten vorgegeben wird, um mindestens eine der anderen Flussraten zu berechnen, so dass die gewünschte Dialysance beibehalten wird. Der nachfolgend aufgezeigte Zusammenhang schließt den Fall ein, dass vor oder während der Behandlung eine bestimmte Dialysance und mindestens eine der Flussraten vorgegeben wird. Wenn während der Behandlung eine bestimmte Dialysance vorgegeben wird, können die relevanten Flussraten berechnet werden, so dass die gewünschte Dialysance beibehalten wird. Diese Berechnung kann immer dann erfolgen, wenn sich eine der Flussraten geändert hat, ohne eine Leitfähigkeitsmessung vornehmen zu müssen.

Der diffusive Anteil der Dialysance $D_{diff}$ berechnet sich nach Gleichung 4:

$$Ddiff = \frac{Qb + \kappa Qs}{Qb - Qf - (1 - \kappa)Qs} \left( \frac{Qb + \kappa Qs}{Qb} D - Qf - Qs \right) \qquad (4),$$

wobei $\kappa=1$ bei Prädilution und $\kappa=0$ bei Postdilution ist.

Der Filterkoeffizient k0A, der als konstant zwischen den beiden Zeitpunkten 1 und 2 angenommen wird, berechnet sich wie folgt:

$$k\,0A = \frac{(Qb + \kappa Qs)Qd}{Qd - Qb - \kappa Qs} \ln \frac{\dfrac{Ddiff}{Qd} - 1}{\dfrac{Ddiff}{Qb + \kappa Qs} - 1} \qquad (5).$$

$$Ddiff = Qb \frac{e^{\gamma} - 1}{e^{\gamma} - \dfrac{Qb}{Qd}}, mit\, \gamma = k0A \frac{Qd - Qb}{QbQd} \qquad (6).$$

Die obigen Gleichungen zeigen den Zusammenhang zwischen den Flüssen Qf, Qs, Qd und Qb sowie den Größen k0A, D und Ddiff.

Nach der Erfindung wird mindestens einer der Flüsse vorgegeben, wobei nach den obigen Gleichungen mindestens eine der anderen Flüsse bestimmt wird, so dass die gewünschte Dialysance D erzielt wird. Die Bestimmung der Flüsse nach den obigen Gleichungen kann mit den bekannten Rechenverfahren zumindest numerisch erfolgen.

Nachfolgend wird unter Bezugnahme auf die Figuren 2a und 2b ein Verfahren zur Bestimmung der Dialysance D kurz beschrieben, das aus der US 2003/0230533 bekannt ist. In den Figuren 2a und 2b ist ein Bereich 250 gestrichelt eingerahmt. Betrachtet man diesen Bereich als eine Art Black-Box Dialysator 1, so können die Formalismen, die sich für die in Fig. 1 gezeigte Anordnung ergeben, auf die

Situation der Hämofiltration übertragen werden. Falls die physikalisch-chemische Eigenschaft eine Konzentration ist, ergeben sich die folgenden Gleichungen:

$$D = (Qf + Qs + Qd) \frac{Cf - Cs}{\alpha Cbi - Cs} \qquad (7).$$

wobei $\alpha$ der Gibbs-Donnan-Koeffizient ist.

$$D = (Qf + Qs + Qd)(1 - \frac{Cf2 - Cf1}{Cs2 - Cs1}) = (Qf + Qs + Qd)(1 - \frac{\Delta Cf}{\Delta Cs}) \qquad (8).$$

Nach einer anfänglichen Bestimmung der Ionen-Dialysance D ist es möglich, weitere Werte der Dialysance für spätere Zeitpunkte zu berechnen, bei denen sich mindestens einer der Flüsse Qs, Qf, Qd oder Qb geändert hat. Dies setzt aber voraus, dass die Flüsse Qs, Qf, Qd oder Qb zu einem Zeitpunkt vor deren Änderung bekannt sind. Die anfängliche Bestimmung der Dialysance bei den Flüssen Qfl, Qs1, Qd1 und Qb1 kann mit den bekannten Verfahren auf der Grundlage einer Leitfähigkeitsmessung erfolgen. Da diese Verfahren zum Stand der Technik gehören, erübrigt sich eine weitere Beschreibung. Ein derartiges Verfahren ist beispielsweise in der EP 0 428 927 A1 oder der US 2003/0230533 im Einzelnen beschrieben.

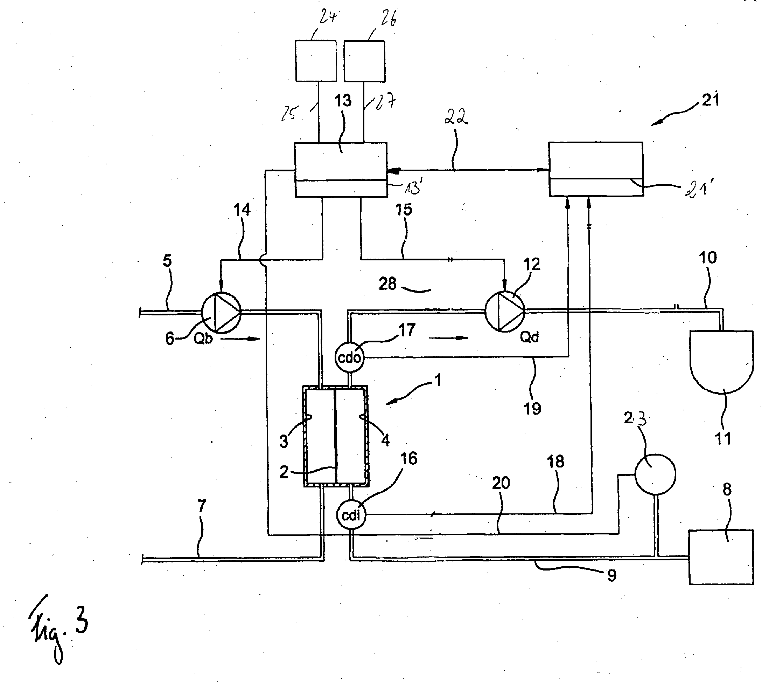

Nachfolgend wird der oben beschriebene Zusammenhang zwischen Clearance oder Dialysance und den einzelnen Flüssen bzw. Flussraten für den Fall der Hämodialyse unter Bezugnahme auf Fig. 3 erläutert.

Die extrakorporale Blutbehandlungsvorrichtung, bei der es sich um eine Hämodialysevorrichtung handelt, verfügt über einen Dialysator 1, der durch eine semipermeable Membran 2 in eine Blutkammer 3 und eine Dialysierflüssigkeitskammer 4 unterteilt ist. Von einem Patienten führt eine arterielle Blutleitung 5, in die eine Blutpumpe 6 geschaltet ist, zu einem Einlass der Blutkammer 3, während von einem Auslass der Blutkammer eine venöse Blutleitung 7 zu dem Patienten führt.

In einer Dialysierflüssigkeitsquelle 8 wird frische Dialysierflüssigkeit bereitgestellt. Von der Dialysierflüssigkeitsquelle 8 führt eine Dialysierflüssigkeitszuführleitung 9 zu einem Einlass der Dialysierflüssigkeitskammer 4 des Dialysators 1, während eine Dialysierflüssigkeitsabführleitung 10 von einem Auslass der Dialysierflüssigkeitskammer zu einem Abfluss 11 führt. In die Dialysierflüssigkeitsabführleitung 10 ist eine Dialysierflüssigkeitspumpe 12 geschaltet.

Die Dialysevorrichtung verfügt über eine Steuereinheit 13, die mit der Blutpumpe 6 und der Dialysierflüssigkeitspumpe 12 über Steuerleitungen 14, 15 verbunden ist. Die Steuereinheit 13 erzeugt Steuersignale zum Betreiben der Blut- und Dialysierflüssigkeitspumpe 6, 12 mit einer vorgegebenen Förderrate, so dass sich in der Blutleitung 5 eine vorgegebene Blutflussrate Qb und in der Dialysierflüssigkeitsleitung 10 eine vorgegebene Dialysierflüssigkeitsrate Qd einstellen.

In der Dialysierflüssigkeitszuführleitung 9 am Einlass der Dialysierflüssigkeitskammer 4 ist ein Leitfähigkeitssensor 16 zur Bestimmung der Dialysierflüssigkeitseingangskonzentration $c_{di}$ einer bestimmten Substanz in der Dialysierflüssigkeit stromauf der Dialysierflüssigkeitskammer und ein Leitfähigkeitssensor 17 in der Dialysierflüssigkeitsabführleitung 10 am Auslass der Dialysierflüssigkeitskammer 4 angeordnet, der die sich während der Dialysebehandlung einstellende Dialysierflüssigkeitsausgangskonzentration $c_{do}$ der betreffenden Substanz in der Dialysierflüssigkeit stromab des Dialysators misst.

Die Messwerte der Leitfähigkeitssensoren 16, 17 werden über Signalleitungen 18, 19 einer Einrichtung 21 zur Bestimmung der Clearance K oder der Dialysance D zugeführt. Über eine zu der Steuereinheit 13 führende Datenleitung 22 empfängt die Einrichtung 21 zur Bestimmung der Clearance oder Dialysance die den Blutfluss Qb bzw. den Dialysierflüssigkeitsfluss Qd vorgebenden Steuersignale der Blutpumpe 6 bzw. der Dialysierflüssigkeitspumpe 12. Andererseits empfängt die Steuereinheit 13 von der Einrichtung 21 über die Datenleitung 22 die mit der Einrichtung 21 bestimmte Clearance oder Dialysance.

Zur Veränderung der Na-Konzentration der Dialysierflüssigkeit stromauf des Dialysators 1 ist eine weitere Einrichtung 23 vorgesehen, mit der die in den Dialysator fließende Dialysierflüssigkeit bezüglich ihrer Zusammensetzung verändert werden kann. Über eine Steuerleitung 20 ist die Einrichtung 23 mit der Steuereinheit 13 verbunden.

Darüber hinaus weist die Dialysevorrichtung eine Eingabeeinheit 24 auf, die mit einer Datenleitung 25 mit der Steuereinheit 13 verbunden ist. Mit der Eingabeeinheit 24 kann eine gewünschte Clearance K oder Dialysance D eingegeben werden. Auch ist eine Eingabe einer gewünschten Blutflussrate $Q_b$ oder Dialysierflüssigkeitsrate $Q_d$ möglich, um entweder die eine oder andere oder beide Größen vorgeben und/oder während der Behandlung verändern zu können. Des weiteren ist eine Speichereinheit 26 vorgesehen, die mit einer Datenleitung 27 ebenfalls mit der Steuereinheit 13 verbunden ist. Die mit der Eingabeeinheit 24 eingegebenen Werte werden in der Speichereinheit 26 gespeichert und können von der Steuereinheit aus der Speichereinheit ausgelesen werden.

Die Dialysevorrichtung erlaubt verschiedene Betriebsarten, die nachfolgend näher beschrieben werden. Aber nicht alle der Betriebsarten setzen die Messung der Dialysance oder der Clearance voraus. Daher kann für diese Betriebsarten auch auf die Einrichtung zur Messung der Clearance oder Dialysance verzichtet werden, die von den mit den Bezugsziffern 21, 23 und 16 und 17 bezeichneten Komponenten gebildet wird.

Die Dialysevorrichtung verfügt noch über weitere Komponenten, z.B. eine Tropfkammer, Sperrorgane etc., die dem Fachmann bekannt sind und der besseren Übersichtlichkeit halber jedoch nicht dargestellt sind. Auch kann die Dialysevorrichtung eine Ultrafiltrationseinrichtung aufweisen.

Der Anwender gibt mit der Eingabeeinheit 24, die beispielsweise über eine Bildschirmeirigabe oder eine Tastatur verfügt, neben verschiedenen anderen Parametern der Hämodialyse die gewünschte Clearance K oder Dialysance D ein. Darüber hinaus ist die Eingabe der Behandlungsdauer T sowie einer gewünschten Blutflussrate Qb und/oder Dialysierflüssigkeitsrate Qd möglich. Die Werte werden in der Speichereinheit 26 gespeichert und können von der Steuereinheit 13 ausgelesen werden.

Die Steuereinheit 13 verfügt über eine Recheneinheit 13', die aus der gewünschten Clearance oder Dialysance sowie der Blutflussrate die Dialysierflüssigkeitsrate berechnet, die erforderlich ist, um die gewünschte Clearance oder Dialysance zu erzielen. Wenn der Anwender nicht die Blutflussrate, sondern die Dialysierflüssigkeitsrate vorgegeben hat, berechnet die Recheneinheit 13' die erforderliche Blutflussrate zur Erzielung der gewünschten Clearance oder Dialysance.

Die Berechnung der erforderlichen Dialysierflüssigkeitsrate Qb bzw. Blutflussrate Qd erfolgt mit dem Koeffizienten k0A auf der Grundlage der folgenden Gleichung:

$$K = Q_b Q_d \frac{1 - \exp\left(-k0A \frac{Q_d - Q_b}{Q_d Q_b}\right)}{Q_d - Q_b \exp\left(-k0A \frac{Q_d - Q_b}{Q_d Q_b}\right)} \tag{9}$$

k0A stellt dabei einen Koeffizienten dar, der im Wesentlichen von der aktiven Oberfläche A der semipermeablen Membran des Dialysators ($m^2$) und dem Diffussionswiderstand R der Membran des Dialysators ($m^2$min/ml = $10^4$ min/cm) abhängt (k0A=A/R).

Während die oben angegebenen Gleichungen den Zusammenhang zwischen Dialysance oder Clearance in allgemeiner Form beschreiben, beschreibt Gleichung 9 den Zusammenhang für den Spezialfall der Hämodialyse. Zur Auflösung der Gleichung werden im Allgemeinen numerische Verfahren herangezogen, die dem Fachmann bekannt sind.

Bei dem Koeffizienten k0A handelt es sich um einen dialysatortypischen Kennwert, der von der Steuereinheit 13 aus der Speichereinheit 26 ausgelesen wird. In der Speichereinheit 26 können eine Vielzahl von Koeffizienten k0A gespeichert sein, die jeweils unterschiedlichen Dialysatortypen zugeordnet sind. Mit der Eingabeeinheit 24 kann der Anwender vor Beginn der Behandlung einen bestimmten Dialysatortyp eingeben, so dass die Steuereinheit 13 den jeweils zugehörigen Koeffizienten aus der Speichereinheit 26 auslesen kann.

Aufgrund des Zusammenhangs zwischen Blutflussrate und Dialysierflüssigkeitsrate sowie Clearance oder Dialysance nach Gleichung (9) führt eine Verringerung der Blutflussrate zu einer Vergrößerung der Dialysierflüssigkeitsrate, um die gewünschte Clearance oder Dialysance zu erzielen. Umgekehrt führt eine Vergrößerung der Blutflussrate zu einer Verringerung der Dialysierflüssigkeitsrate, um die vorgegebene Clearance oder Dialysance zu erzielen. Wenn hingegen nicht die Blutflussrate, sondern die Dialysierflüssigkeitsrate verändert wird, führt eine Vergrößerung der Dialysierflüssigkeitsrate zu einer Verringerung der Blutflussrate bzw. eine Verringerung der Dialysierflüssigkeitsrate zu einer Vergrößerung der Blutflussrate.

Bei einer Änderung der Dialysierflüssigkeitsrate oder Blutflussrate während der Behandlung, die in einzelnen Schritten oder auch kontinuierlich erfolgen kann, wird die Blutflussrate bzw. Dialysierflüssigkeitsrate von der Steuereinheit 13 immer derart angepasst, dass die gewünschte Clearance oder Dialysance innerhalb der vorgegebenen Behandlungszeit beibehalten wird.

Die Steuereinheit 13 berücksichtigt bei der Steuerung der Förderrate der Blutpumpe 6 bzw. Dialysierflüssigkeitspumpe 12, dass bestimmte minimale oder maximale Flussraten für den Blutfluss bzw. Dialysierflüssigkeitsfluss nicht unterschritten oder überschritten werden. Insbesondere der Blutfluss sollte einen bestimmten oberen Grenzwert nicht überschreiten, der von dem Gefäßzugang abhängig ist. Für den Fall, dass die Erzielung der gewünschten Clearance oder Dialysance ein Über- bzw. Unterschreiten der jeweiligen Flussraten von Blutfluss- bzw. Dialysierflüssigkeitsfluss erforderlich machen sollte, signalisiert die Steuereinheit 13 diesen Störfall dem Anwender. Hierzu kann beispielsweise eine nicht dargestellte Alarmeinrichtung vorgesehen sein, die einen akustischen und/oder optischen Alarm gibt. Die Steuereinheit 13 kann dann eine längere bzw. kürzere Behandlungszeit vorgeben, um die Einstellung der Flussrate innerhalb der vorgegebenen Grenzen von Blutfluss und Dialysierflüssigkeitsfluss vornehmen zu können.

Nachfolgend wird eine alternative Ausführungsform der Steuerungsvorrichtung beschrieben, die von der Einrichtung zum Messen der Dialysance Gebrauch macht.

Die Messung der Dialysance beruht darauf, dass die Dialysierflüssigkeitseingangskonzentration $c_{di}$, beispielsweise die Na-Konzentration der Dialysierflüssigkeit, stromauf des Dialysators 1 mittels der Einrichtung 23 zur Veränderung der Zusammensetzung der Dialysierflüssigkeit kurzzeitig verändert wird, und die Dialysierflüssigkeitseingangs- $c_{di}$ und - ausgangskonzentration $c_{do}$ mittels der Messeinrichtungen 16, 17 stromauf und stromab des Dialysators gemessen werden. Die Messwerte werden von der Einrichtung 21 zur Bestimmung der Clearance oder Dialysance verarbeitet, die über eine Recheneinheit 21' verfügt, um die Clearance oder Dialysance zu berechnen.

Die Berechnung der Clearance K oder Dialysance D kann für eine bestimmte Blutflussrate und Dialysierflüssigkeitsrate grundsätzlich nach den Gleichungen (1) bis (3) erfolgen. Dieses Verfahren zur Bestimmung der Clearance oder Dialysance ist in der EP 0 428 927 A1 im Einzelnen beschrieben, auf die ausdrücklich Bezug genommen wird.

Ein weiteres Verfahren zur Bestimmung der Clearance oder der Dialysance, das sich durch eine besonders kurze Messzeit auszeichnet, ist aus der US 6,702,774 B1 bekannt, auf die ebenfalls zum Zwecke der Offenbarung ausdrücklich Bezug genommen wird. Aufgrund der kurzen Messzeiten ist der Anwendung dieses Verfahrens bei der erfindungsgemäßen Steuerungsvorrichtung der Vorzug zu geben.

Die Steuereinheit 13 steuert die Einrichtung 21 zur Bestimmung der Clearance oder Dialysance zu Beginn der Behandlung an, so dass die Einrichtung 21 bei der zu Beginn der Behandlung vorgegebenen Blutflussrate und Dialysierflüssigkeitsrate die Clearance oder Dialysance bestimmt. Der ermittelte Wert für die Clearance oder Dialysance wird daraufhin von der Steuereinheit ausgelesen, die auf der Grundlage der Gleichung 9 den Koeffizienten k0A berechnet, der dann für die weitere Berechnung der Flussraten nach Gleichung 9 zur Verfügung steht. Diese Ausführungsform hat den Vorteil, dass der Dialysatortyp nicht mit der Eingabeeinheit eingegeben werden muss und eine Tabelle mit einer Zuordnung von verschiedenen Koeffizienten für unterschiedliche Dialysatortypen nicht in der Speichereinheit abgelegt sein muss.

Eine weitere alternative Ausführungsform sieht vor, dass die gewünschte Clearance oder Dialysance nicht mit der Eingabeeinheit 24 eingegeben, sondern zu Beginn der Behandlung von der Steuereinheit 13 vorgegeben wird. Die Steuereinheit 13 kann als Clearance oder Dialysance den Wert vorgeben, den die Einrichtung 21 zur Bestimmung der Clearance oder Dialysance beispielsweise zu Beginn der Behandlung ermittelt hat.

Der unter Bezugnahme auf die Hämodialyse beschriebene Formalismus lässt sich auch auf die Hämofiltration anwenden. Daher unterscheidet sich die Steuerungsvorrichtung für eine Hämofiltrationsvorrichtung von der oben beschriebenen Steuerungsvorrichtung nur dadurch, dass neben der Blutflussrate noch die Ultrafiltrationsrate und/oder die Substituatrate bei der Auswertung Berücksichtigung findet, die Dialysierflüssigkeitsrate aber nicht berücksichtigt wird. Bei der Hämodiafiltration finden neben der Blutflussrate und Dialysierflüssigkeitsrate die Ultrafiltrationsrate und/oder Substituatrate Berücksichtigung. Wenn eine der Flussraten, beispielsweise die Dialysierflüssigkeitsrate geändert wird, berechnet die Recheneinheit 13' der Steuereinheit 13 auf der Grundlage des in den Gleichungen 4 bis 6 beschriebenen Zusammenhangs eine der anderen Flussraten, beispielsweise die Blutflussrate oder Ultrafiltrationsrate oder Substituatrate, bei der die gewünschte Clearance oder Dialysance während der Blutbehandlung sichergestellt ist, ohne jedoch fortlaufend Leitfähigkeitsmessungen vornehmen zu müssen. Dabei ist es lediglich erforderlich, die Clearance oder Dialysance für einen Satz von Flüssen Qfl und/oder Qs1 und/oder Qd1 und/oder Qb1 einmal zu messen, um bei einer Änderung einer Flussrate eine andere Flussrate allein auf der Grundlage einer Berechnung der Größen entsprechend anzupassen. Diese Messung erfolgt mit der Einrichtung 21 zu Bestimmung der Clearance oder Dialysance mit der Leitfähigkeitsmessung nach der kurzzeitigen Änderung der Dialysierflüssigkeits-bzw. Substituatzusammensetzung. Die Berechnung der jeweiligen anderen Flussrate, die der Änderung der einen Flussrate zur Gewährleistung der vorgegebenen Clearance oder Dialysance entgegenwirken soll, wird immer dann vorgenommen, wenn die eine Flussrate geändert worden ist.

## Patentansprüche

1. Vorrichtung zur Steuerung einer extrakorporalen Blutbehandlungsvorrichtung,
   wobei die Blutbehandlungsvorrichtung aufweist:

   eine Austauscheinheit, die durch eine semipermeable Membran in eine erste Kammer und eine zweite Kammer unterteilt ist, wobei die erste Kammer Teil eines extrakorporalen Blutkreislaufs ist, in dem eine Blutpumpe zum Fördern von Blut mit einer bestimmten Blutflussrate Qb angeordnet ist,
   und/oder Mittel zum Fördern von Dialysierflüssigkeit mit einer bestimmten Dialysierflüssigkeitsrate Qd durch die zweite Kammer der Austauscheinheit,
   und/oder Mittel zum Zuführen von Substituat mit einer bestimmten Substituatrate Qs in den extrakorporalen Blutkreislauf,
   und/oder Mittel zum Entziehen von Ultrafiltrat aus der ersten Kammer durch die semipermeable Membran in die zweite Kammer der Austauscheinheit mit einer Flüssigkeitsrate, die der Summe der Substituatrate Qs und einer bestimmten Ultrafiltrationsrate Qf entspricht,

   wobei die Vorrichtung zur Steuerung der Blutbehandlungsvorrichtung aufweist:

   eine Steuereinheit (13) zum Erzeugen eines Steuersignals zum Einstellen der Förderrate der Blutpumpe auf eine bestimmte Blutflussrate Qb und zum Erzeugen eines Steuersignals zum Einstellen mindestens einer bestimmten Flussrate aus der Gruppe der Flussarten von Dialysierflüssigkeitsrate Qd, Ultrafiltrationsrate Qf und Substituatrate Qs, einer Speichereinheit (26) zum Speichern einer vorgegebenen Clearance K oder Dialysance D,
   **dadurch gekennzeichnet, dass**

eine Recheneinheit (13') vorgesehen ist, die derart ausgebildet ist,
dass bei mindestens einer vorgegebenen Flussrate aus der Gruppe der Flussraten von Blutflussrate Qb, Dialysierflüssigkeitsrate Qd, Ultrafiltratrate Qf und Substituatrate Qs mindestens eine der jeweils anderen Flussraten aus der Gruppe der Flussraten von Blutflussrate Qb, Dialysierflüssigkeitsrate Qd, Ultrafiltratrate Qf und Substituatrate Qs allein auf der Grundlage einer vorgegebenen Abhängigkeit der Clearance K oder Dialysance D von den Flussraten berechnet wird, bei der die vorgegebene Clearance K oder Dialysance D beibehalten wird.

2. Vorrichtung nach Anspruch 1, **dadurch gekennzeichnet, dass** die Recheneinheit (13') die mindestens eine der jeweils anderen Flussraten aus der Gruppe der Flussraten von Blutflussrate Qb, Dialysierflüssigkeitsrate Qd, Ultrafiltratrate Qf und Substituatrate Qs bei einer Änderung der mindestens einen von der Steuereinheit (13) vorgegebenen Flussrate zumindest für einen Zeitabschnitt der Blutbehandlung fortlaufend berechnet.

3. Vorrichtung nach Anspruch 1, **dadurch gekennzeichnet, dass** die Steuereinheit (13) mit der Recheneinheit (13') derart zusammenwirkt, dass die Steuereinheit (13) Steuersignale zum Einstellen der berechneten mindestens einen Flussrate aus der Gruppe der Flussarten von Blutflussrate Qb, Dialysierflüssigkeitsrate Qd, Ultrafiltratrate Qf und Substituatrate Qs erzeugt, so dass sich die mindestens eine Flussrate einstellt.

4. Vorrichtung zur Steuerung einer Hämodialysevorrichtung, wobei die Hämodialysevorrichtung aufweist:

   einen Dialysator, der durch eine semipermeable Membran in eine Blutkammer und eine Dialysierflüssigkeitskammer unterteilt ist, und
   eine Blutpumpe zum Fördern von Blut durch die Blutkammer mit einer bestimmten Blutflussrate und eine Dialysierflüssigkeitspumpe zum Fördern von Dialysierflüssigkeit durch die Dialysierflüssigkeitskammer mit einer bestimmten Dialysierflüssigkeitsrate,

   wobei die Vorrichtung zur Steuerung der Hämodialysevorrichtung aufweist:

   eine Steuereinheit (13) zum Erzeugen eines Steuersignals zum Einstellen der Förderrate der Blutpumpe auf eine bestimmte Blutflussrate Qb und zum Erzeugen eines Steuersignals zum Einstellen der Förderrate der Dialysierflüssigkeitspumpe auf eine bestimmte Dialysierflüssigkeitsrate Qd, und
   eine Speichereinheit (26) zum Speichern einer vorgegebenen Clearance K oder Dialysance D,
   **dadurch gekennzeichnet, dass**
   eine Recheneinheit (13') vorgesehen ist, die derart ausgebildet ist,
   dass für eine vorgegebene Blutflussrate Qb allein auf der Grundlage einer vorgegebenen Abhängigkeit der Clearance oder Dialysance von der Blutflussrate und Dialysierflüssigkeitsrate die Dialysierflüssigkeitsrate Qd ermittelt wird, bei der mit der vorgegebenen Blutflussrate eine vorgegebene Clearance K oder Dialysance beibehalten wird, und/oder
   dass für eine vorgegebene Dialysierflüssigkeitsrate Qd allein auf der Grundlage einer vorgegebenen Abhängigkeit der Clearance oder Dialysance von der Blutflussrate und Dialysierflüssigkeitsrate die Blutflussrate Qb ermittelt wird, bei der mit der vorgegebenen Dialysierflüssigkeitsrate eine vorgegebene Clearance K oder Dialysance D beibehalten wird.

5. Vorrichtung nach Anspruch 4, **dadurch gekennzeichnet, dass** die Recheneinheit (13') die Blutflussrate Qb oder Dialysierflüssigkeitsrate Qd bei einer Änderung der von der Steuereinheit vorgegebenen Dialysierflüssigkeitsrate Qd oder Blutflussrate Qb zumindest für einen Zeitabschnitt der Blutbehandlung fortlaufend berechnet.

6. Vorrichtung nach einem der Ansprüche 1 bis 5, **dadurch gekennzeichnet, dass**
   die Steuereinheit (13) Steuersignale zum Betreiben der Dialysierflüssigkeitspumpe mit einer Förderrate erzeugt, so dass sich die ermittelte Dialysierflüssigkeitsrate Qd einstellt, und/oder
   die Steuereinheit (13) Steuersignale zum Betreiben der Blutpumpe mit einer Förderrate erzeugt, so dass sich die ermittelte Blutflussrate Qb einstellt.

7. Vorrichtung nach einem der Ansprüche 1 bis 6, **dadurch gekennzeichnet, dass** eine Eingabeeinheit (24) zur Eingabe einer gewünschten Clearance K oder Dialysance D mit der Speichereinheit (26) derart zusammenwirkt, dass die eingegebene Clearance oder Dialysance in der Speichereinheit gespeichert wird, wobei die Steuereinheit (13) die eingegebene Clearance oder Dialysance als vorgegebene Clearance oder Dialysance übernimmt.

8. Vorrichtung nach einem der Ansprüche 1 bis 7, **dadurch gekennzeichnet, dass** eine Eingabeeinheit (24) zur Eingabe einer gewünschten Blutflussrate und/oder Dialysierflüssigkeitsrate mit der Steuereinheit (13) derart zusammenwirkt, dass Steuersignale zum Betreiben der Blutpumpe und/oder Dialysierflüssigkeitspumpe mit der eingegebenen Blutflussrate Qb und/oder Dialysierflüssigkeitsrate Qd erzeugt werden.

9. Vorrichtung nach einem der Ansprüche 1 bis 8, **dadurch gekennzeichnet, dass** die Recheneinheit (13') derart ausgebildet ist, dass bei einer Vergrößerung der Blutflussrate Qb die Dialysierflüssigkeitsrate Qd soweit verringert und bei einer Verringerung der Blutflussrate Qb die Dialysierflüssigkeitsrate Qd soweit vergrößert wird, dass die vorgegebene Clearance K oder Dialysance D beibehalten wird, und/oder dass bei einer Vergrößerung der Dialysierflüssigkeitsrate Qd die Blutflussrate Qb soweit verringert und bei einer Verringerung der Dialysierflüssigkeitsrate Qd die Blutflussrate Qb soweit vergrößert wird, dass die vorgegebene Clearance oder Dialysance beibehalten wird.

10. Vorrichtung nach einem der Ansprüche 4 bis 9, **dadurch gekennzeichnet, dass** der Zusammenhang zwischen der vorgegebenen Clearance K oder Dialysance D einerseits und der Blutflussrate Qb und Dialysierflüssigkeitsrate Qb andererseits mit der folgenden Gleichung beschrieben wird:

$$K = Q_b Q_d \frac{1 - \exp\left(-k0A \frac{Q_d - Q_b}{Q_d Q_b}\right)}{Q_d - Q_b \exp\left(-k0A \frac{Q_d - Q_b}{Q_d Q_b}\right)}$$

wobei k0A ein Koeffizient ist.

11. Vorrichtung nach einem der Anspruch 10, **dadurch gekennzeichnet, dass** in der Speichereinheit (26) für unterschiedliche Typen von Dialysatoren verschiedene Werte für den Koeffizienten k0A gespeichert sind, die von der Steuereinheit (13) übernommen werden.

12. Vorrichtung nach Anspruch 11, **dadurch gekennzeichnet, dass** die Steuereinheit (13) mit einer Messeinheit (21) derart zusammenwirkt, dass für eine von der Steuereinheit vorgegebene Blutflussrate Qb und Dialysierflüssigkeitsrate Qd mit der von der Messeinheit ermittelten Clearance K oder Dialysance D der Koeffizient k0A berechnet wird.

13. Blutbehandlungsvorrichtung mit
einer Austauscheinheit, die durch eine semipermeable Membran in eine erste Kammer und eine zweite Kammer unterteilt ist, wobei die erste Kammer Teil eines extrakorporalen Blutkreislaufs ist, in dem eine Blutpumpe zum Fördern von Blut mit einer bestimmten Blutflussrate Qb angeordnet ist,
und/oder Mitteln zum Fördern von Dialysierflüssigkeit mit einer bestimmten Dialysierflüssigkeitsrate Qd durch die zweite Kammer der Austauscheinheit,
und/oder Mitteln zum Zuführen von Substituat mit einer bestimmten Substituatrate Qs in den extrakorporalen Blutkreislauf,
und/oder Mitteln zum Entziehen von Ultrafiltrat aus der ersten Kammer durch die semipermeable Membran in die zweite Kammer der Austauscheinheit mit einer Flüssigkeitsrate, die der Summe der Substituatrate Qs und einer bestimmten Ultrafiltrationsrate Qf entspricht,
**dadurch gekennzeichnet, dass** die Blutbehandlungsvorrichtung eine Steuervorrichtung nach einem der Ansprüche 1 bis 3 aufweist.

14. Hämodialysevorrichtung mit
einem Dialysator (1), der durch eine semipermeable Membran (2) in eine Blutkammer (3) und eine Dialysierflüssigkeitskammer (4) unterteilt ist,
einer Blutpumpe (6) zum Fördern von Blut durch die Blutkammer (3) mit einer bestimmten Blutflussrate Qb und einer Dialysierflüssigkeitspumpe (12) zum Fördern von Dialysierflüssigkeit durch die Dialysierflüssigkeitskammer (4) mit einer bestimmten Dialysierflüssigkeitsrate Qd,
**dadurch gekennzeichnet, dass** die Hämodialysevorrichtung eine Steuervorrichtung nach einem der Ansprüche 4 bis 12 aufweist.

15. Verfahren zum Steuern einer extrakorporalen

Blutbehandlungsvorrichtung, wobei die Blutbehandlungsvorrichtung aufweist:

eine Austauscheinheit, die durch eine semipermeable Membran in eine erste Kammer und eine zweite Kammer unterteilt ist, wobei die erste Kammer Teil eines extrakorporalen Blutkreislaufs ist, in dem eine Blutpumpe zum Fördern von Blut mit einer bestimmten Blutflussrate Qb angeordnet ist,
und/oder Mittel zum Fördern von Dialysierflüssigkeit mit einer bestimmten Dialysierflüssigkeitsrate Qd durch die zweite Kammer der Austauscheinheit,
und/oder Mittel zum Zuführen von Substituat mit einer bestimmten Substituatrate Qs in den extrakorporalen Blutkreislauf,
und/oder Mittel zum Entziehen von Ultrafiltrat aus der ersten Kammer durch die semipermeable Membran in die zweite Kammer der Austauscheinheit mit einer Flüssigkeitsrate, die der Summe der Substituatrate Qs und einer bestimmten Ultrafiltrationsrate Qf entspricht,

mit folgenden Verfahrensschritten:

Speichern einer vorgegebenen Clearance K oder Dialysance D,
Berechnen bei einer vorgegebenen Flussrate aus der Gruppe der Flussraten von Blutflussrate Qb, Dialysierflüssigkeitsrate Qd, Ultrafiltratrate $Q_f$ und Substituatrate Qs mindestens eine der jeweils anderen Flussraten aus der Gruppe der Flussarten von Blutflussrate Qb, Dialysierflüssigkeitsrate Qd, Ultrafiltratrate Qf und Substituatrate Qs allein auf der Grundlage einer vorgegebenen Abhängigkeit der Clearance K oder Dialysance D von den Flussraten, bei der die vorgegebene Clearance K oder Dialysance D beibehalten wird.

16. Verfahren nach Anspruch 15, **dadurch gekennzeichnet, dass** die mindestens eine der jeweils anderen Flussraten aus der Gruppe der Flussraten von Blutflussrate Qb, Dialysierflüssigkeitsrate Qd, Ultrafiltratrate Qf und Substituatrate Qs bei einer Änderung der mindestens einen vorgegebenen Flussrate zumindest für einen Zeitabschnitt der Blutbehandlung fortlaufend berechnet wird.

17. Verfahren nach Anspruch 16, **dadurch gekennzeichnet, dass** die berechnete mindestens eine Flussrate aus der Gruppe der Flussraten von Blutflussrate Qb, Dialysierflüssigkeitsrate Qd, Ultrafiltratrate Qf und Substituatrate Qs eingestellt wird.

18. Verfahren zum Steuern einer Hämodialysevorrichtung, wobei die Hämodialysevorrichtung aufweist:

einen Dialysator, der durch eine semipermeable Membran in eine Blutkammer und eine Dialysierflüssigkeitskammer unterteilt ist, und
eine Blutpumpe zum Fördern von Blut durch die Blutkammer mit einer bestimmten Blutflussrate Qb und eine Dialysierflüssigkeitspumpe zum Fördern von Dialysierflüssigkeit durch die Dialysierflüssigkeitskammer mit einer bestimmten Dialysierflüssigkeitsrate Qd,

mit folgenden Verfahrenschritten:

Speichern einer vorgegebenen Clearance K oder Dialysance D,
Ermitteln für eine vorgegebene Blutflussrate Qb allein auf der Grundlage einer vorgegebenen Abhängigkeit der Clearance oder Dialysance von der Blutflussrate und Dialysierflüssigkeitsrate die Dialysierflüssigkeitsrate Qd, bei der mit der vorgegebenen Blutflussrate eine vorgegebene Clearance K oder Dialysance D beibehalten wird, und/oder
Ermitteln für eine vorgegebene Dialysierflüssigkeitsrate Qd allein auf der Grundlage einer vorgegebenen Abhängigkeit der Clearance oder Dialysance von der Blutflussrate und Dialysierflüssigkeitsrate die Blutflussrate Qb, bei der mit der vorgegebenen Dialysierflüssigkeitsrate eine vorgegebene Clearance K oder Dialysance D beibehalten wird.

19. Verfahren nach Anspruch 18, **dadurch gekennzeichnet, dass** die Blutflussrate Qb oder Dialysierflüssigkeitsrate Qd bei einer Änderung der vorgegebenen Dialysierflüssigkeitsrate Qd oder Blutflussrate Qb zumindest für einen Zeitabschnitt der Blutbehandlung fortlaufend berechnet wird.

20. Verfahren nach Anspruch 18 oder 19, **dadurch gekennzeichnet, dass** die ermittelte Dialysierflüssigkeitsrate Qd oder Blutflussrate Qb eingestellt werden.

**21.** Verfahren nach einem der Ansprüche 18 bis 20, **dadurch gekennzeichnet, dass** eine gewünschte Clearance K oder Dialysance D eingegeben wird, wobei die eingegebene Clearance oder Dialysance die vorgegebene Clearance oder Dialysance ist.

**22.** Verfahren nach einem der Ansprüche 18 bis 21, **dadurch gekennzeichnet, dass** bei einer Vergrößerung der Blutflussrate Qb die Dialysierflüssigkeitsrate Qd soweit verringert und bei einer Verringerung der Blutflussrate die Dialysierflüssigkeitsrate soweit vergrößert wird, dass die vorgegebene Clearance K oder Dialysance D beibehalten wird, und/oder dass bei einer Vergrößerung der Dialysierflüssigkeitsrate Qd die Blutflussrate Qb soweit verringert und bei einer Verringerung der Dialysierflüssigkeitsrate die Blutflussrate soweit vergrößert wird, dass die vorgegebene Clearance oder Dialysance beibehalten wird.

**23.** Verfahren nach Anspruch 22, **dadurch gekennzeichnet, dass** der Zusammenhang zwischen der vorgegebenen Clearance K oder Dialysance D einerseits und der Blutflussrate Qb und Dialysierflüssigkeitsrate Qd andererseits mit der folgenden Gleichung beschrieben wird:

$$K = Q_b Q_d \frac{1 - \exp\left(-k0A\dfrac{Q_d - Q_b}{Q_d Q_b}\right)}{Q_d - Q_b \exp\left(-k0A\dfrac{Q_d - Q_b}{Q_d Q_b}\right)}$$

k0A ein Koeffizient ist.

**24.** Verfahren nach Anspruch 23, **dadurch gekennzeichnet, dass** für unterschiedliche Typen von Dialysatoren verschiedene Werte für den Koeffizienten k0A aus einer Speichereinheit ausgelesen werden.

**25.** Verfahren nach Anspruch 24, **dadurch gekennzeichnet, dass** bei einer vorgegebenen Blutflussrate Qb und einer vorgegebenen Dialysierflüssigkeitsrate Qd die Clearance K oder Dialysance D gemessen und der Koeffizient k0A berechnet wird.

**26.** Computerprogrammprodukt für eine Steuervorrichtung einer extrakorporalen Blutbehandlungsvorrichtung, **dadurch gekennzeichnet, dass** die Verfahrenschritte nach einem der Ansprüche 18 bis 25 ausgeführt werden, wenn die Steuervorrichtung mit dem Programm betrieben wird.

**Claims**

**1.** Device for controlling an extracorporeal blood treatment device,
the blood treatment device comprising:

an exchange unit, which is subdivided into a first chamber and a second chamber by a semipermeable membrane, the first chamber being part of an extracorporeal blood circuit in which a blood pump for conveying blood at a particular blood flow rate Qb is arranged,
and/or means for conveying dialysate through the second chamber of the exchange unit at a particular dialysate rate Qd
and/or means for supplying substitution fluid to the extracorporeal blood circuit at a particular substitution fluid rate Qs,
and/or means for withdrawing ultrafiltrate from the first chamber through the semipermeable membrane into the second chamber of the exchange unit at a fluid rate which corresponds to the total of the substitution fluid rate Qs and a particular ultrafiltration rate Qf,

the device for controlling the blood treatment device comprising:

a control unit (13) for generating a control signal for setting the feed rate of the blood pump to a particular blood flow rate Qb and for generating a control signal for setting at least one particular flow rate from the set of flow types of dialysate rate Qd ultrafiltration rate Qf and substitution fluid rate Qs,

a memory unit (26) for storing a preset clearance K or dialysance D,

**characterised in that**

a central processing unit (13') is provided, which is formed in such a way that

at at least one preset flow rate from the set of flow rates of blood flow rate Qb, dialysate rate Qd ultrafiltrate rate Of and substitution fluid rate Qs, at least one of the respective other flow rates from the set of flow rates of blood flow rate Qb, dialysate rate Qd ultrafiltrate rate Of and substitution fluid rate Qs is calculated purely on the basis of a preset dependence of the clearance K or dialysance D on the flow rates, for which the preset clearance K or dialysance D is maintained.

2. Device according to claim 1, **characterised in that**, in the event of a change in the at least one flow rate preset by the control unit (13), the central processing unit (13') calculates the at least one of the respective other flow rates from the set of flow rates of blood flow rate Qb, dialysate rate Qd, ultrafiltrate rate Of and substitution fluid rate Qs continuously, at least for a time portion of the blood treatment.

3. Device according to claim 1, **characterised in that** the control unit (13) cooperates with the central processing unit (13') in such a way that the control unit (13) generates control signals for setting the calculated at least one flow rate from the set of flow types of blood flow rate Qb, dialysate rate Qd, ultrafiltrate rate Of and substitution fluid rate Qs, in such a way that the at least one flow rate is set.

4. Device for controlling a haemodialysis device, the haemodialysis device comprising:

a dialyser, which is subdivided into a blood chamber and a dialysate chamber by a semipermeable membrane, and

a blood pump for conveying blood through the blood chamber at a particular blood flow rate and a dialysate pump for conveying dialysate through the dialysate chamber at a particular dialysate rate,

the device for controlling the haemodialysis device comprising:

a control unit (13) for generating a control signal for adjusting the feed rate of the blood pump to a particular blood flow rate Qb and for generating a control signal for setting the feed rate of the dialysate pump to a particular dialysate rate Qd, and

a memory unit (26) for storing a preset clearance K or dialysance D,

**characterised in that**

a central processing unit (13') is provided, which is formed in such a way that,

for a predetermined blood flow rate Qb, the dialysate rate Qd at which a predetermined clearance K or dialysance is maintained at the preset blood flow rate is determined purely on the basis of a predetermined dependence of the clearance or dialysance on the blood flow rate and dialysate rate, and/or

for a preset dialysate rate Qd, the blood flow rate Qb at which a predetermined clearance K or dialysance D is maintained at the preset dialysate rate is determined purely on the basis of a preset dependence of the clearance or

dialysance on the blood flow rate and dialysate rate.

5. Device according to claim 4, **characterised in that**, in the event of a change in the dialysate rate Qd or blood flow rate Qb preset by the control unit, the central processing unit (13') calculates the blood flow rate Qb or dialysate rate Qd continuously, at least for a time portion of the blood treatment.

6. Device according to any of claims 1 to 5, **characterised in that**

the control unit (13) generates control signals for operating the dialysate pump at a feed rate such that the determined dialysate rate Qd is set, and/or

the control unit (13) generates control signals for operating the blood pump at a feed rate such that the determined blood flow rate Qb is set.

7. Device according to any of claims 1 to 6, **characterised in that** an input unit (24) for inputting a desired clearance K or dialysance D cooperates with the memory unit (26) in such a way that the input clearance or dialysance is stored in the memory unit, the control unit (13) taking on the input clearance or dialysance as the preset clearance or dialysance.

8. Device according to any of claims 1 to 7, **characterised in that** an input unit (24) for inputting a desired blood flow rate and/or dialysate rate cooperates with the control unit (13) in such a way that control signals are generated for operating the blood pump and/or dialysate pump at the input blood flow rate Qb and/or dialysate rate Qd.

9. Device according to any of claims 1 to 8, **characterised in that** the central processing unit (13') is formed in such a way that, in the event of an increase in the blood flow rate Qb, the dialysate rate Qd is decreased to such an extent, and in the event of a decrease in the blood flow rate Qb the dialysate rate Qd is increased to such an extent, that the preset clearance K or dialysance D is maintained, and/or that in the event of an increase in the dialysate rate Qd the blood flow rate Qb is decreased to such an extent, and in the event of a decrease in the dialysate rate Qd the blood flow rate Qb is increased to such an extent, that the preset clearance K or dialysance D is maintained.

10. Device according to any of claims 4 to 9, **characterised in that** the relationship between the preset clearance K or dialysance D on the one hand and the blood flow rate Qb and dialysate rate Qb on the other hand is described by the following equation:

$$K = Q_b Q_d \frac{1 - \exp\left(-k0A \dfrac{Q_d - Q_b}{Q_d Q_b}\right)}{Q_d - Q_b \exp\left(-k0A \dfrac{Q_d - Q_b}{Q_d Q_b}\right)}$$

where k0A is a coefficient.

11. Device according to claim 10, **characterised in that** different values of the coefficient k0A for different types of dialysers are stored in the memory unit (26), and are taken on by the control unit (13).

12. Device according to claim 11, **characterised in that** the control unit (13) cooperates with a measurement unit (21) in such a way that, for a blood flow rate Qb and dialysate rate Qd preset by the control unit, the coefficient k0A is calculated using the clearance or dialysance D determined by the measurement unit.

13. Blood treatment device, comprising
an exchange unit, which is subdivided into a first chamber and a second chamber by a semipermeable membrane, the first chamber being part of an extracorporeal blood circuit in which a blood pump for conveying blood at a particular blood flow rate Qb is arranged,
and/or means for conveying dialysate at a particular dialysate rate Qd through the second chamber of the exchange unit,
and/or means for supplying substitution fluid to the extracorporeal blood circuit at a particular substitution fluid rate Qs, and/or means for withdrawing ultrafiltrate from the first chamber through the semipermeable membrane into the second chamber of the exchange unit at a fluid rate which corresponds to the total of the substitution fluid rate Qs and a particular ultrafiltration rate Qf,
**characterised in that** the blood treatment device comprises a control device according to any of claims 1 to 3.

14. Haemodialysis device, comprising
a dialyser (1), which is subdivided into a blood chamber (3) and a dialysate chamber (4) by a semipermeable membrane (2),
a blood pump (6) for conveying blood through the blood chamber (3) at a particular blood flow rate Qb and a dialysate pump (12) for conveying dialysate through the dialysate chamber (4) at a particular dialysate rate Qd,
**characterised in that** the haemodialysis device comprises a control device according to any of claims 4 to 12.

15. Method for controlling an extracorporeal blood treatment device, the blood treatment device comprising:

an exchange unit, which is subdivided into a first chamber and a second chamber by a semipermeable membrane, the first chamber being part of an extracorporeal blood circuit in which a blood pump for conveying blood at a particular blood flow rate Qb is arranged,
and/or means for conveying dialysate through the second chamber of the exchange unit at a particular dialysate rate Qd,
and/or means for supplying substitution fluid to the extracorporeal blood circuit at a particular substitution fluid rate Qs,
and/or means or withdrawing ultrafiltrate from the first chamber through the semipermeable membrane into the second chamber of the exchange unit at a fluid rate which corresponds to the total of the substitution fluid rate Qs and a particular ultrafiltration rate Qf,

comprising the following method steps:

storing a preset clearance K or dialysance D,
calculating, for a preset flow rate from the set of flow rates of blood flow rate Qb, dialysate rate Qd, ultrafiltrate rate Qf and substitution fluid rate Qs, at least one of the respective other flow rates from the set of flow types of blood flow rate Qb, dialysate rate Qd, ultrafiltrate rate Qf and substitution fluid rate Qs, purely on the basis of a preset dependence of the clearance K or dialysance D on the flow rate at which the preset clearance K or dialysance D is maintained.

16. Method according to claim 15, **characterised in that**, in the event of a change in the at least one preset flow rate, the at least one of the respective other flow rates from the set of flow rates of blood flow rate Qb, dialysate rate Qd, ultrafiltrate rate Qf and substitution fluid rate Qs is calculated continuously, at least for a time portion of the blood treatment.

17. Method according to claim 16, **characterised in that** the calculated at least one flow rate from the set of flow rates of blood flow rate Qb, dialysate rate Qd, ultrafiltrate rate Qf and substitution fluid rate Qs is set.

18. Method for controlling a haemodialysis device, the haemodialysis device comprising:

a dialyser, which is subdivided into a blood chamber and a dialysate chamber by a semipermeable membrane, and
a blood pump for conveying blood through the blood chamber at a particular blood flow rate Qb and a dialysate pump for conveying dialysate through the dialysate chamber at a particular dialysate rate Qd,

comprising the following method steps:

storing a predetermined clearance K or dialysance D,
determining, for a preset blood flow rate Qb, the dialysate rate Qd at which a preset clearance K or dialysance D is maintained at the preset blood flow rate, purely on the basis of a predetermined dependence of the clearance or dialysance on the blood flow rate and dialysate rate, and/or
determining, for a preset dialysate rate Qd, the blood flow rate Qb at which a preset clearance K or dialysance D is maintained at the preset dialysate rate, purely on the basis of a predetermined dependence of the clearance or dialysance on the blood flow rate and dialysate rate.

19. Method according to claim 18, **characterised in that**, in the event of a change in the preset dialysate rate Qd or blood flow rate Qb, the blood flow rate Qb or dialysate rate Qd is calculated continuously, at least for a time portion of the blood treatment.

20. Method according to either claim 18 or claim 19, **characterised in that** the determined dialysate rate Qd or blood flow rate Qb is set.

21. Method according to any of claims 18 to 20, **characterised in that** a desired clearance K or dialysance D is input, the input clearance or dialysance being the preset clearance or dialysance.

22. Method according to any of claims 18 to 21, **characterised in that**, in the event of an increase in the blood flow rate Qb, the dialysate rate Qd is decreased to such an extent, and in the event of a decrease in the blood flow rate the dialysate rate is increased to such an extent, that the preset clearance K or dialysance D is maintained, and/or that, in the event of an increase in the dialysate rate Qd the blood flow rate Qb is decreased to such an extent, and in the event of a decrease in the dialysate rate the blood flow rate is increased to such an extent, that the preset clearance K or dialysance D is maintained.

23. Method according to claim 22, **characterised in that** the relationship between the preset clearance K or dialysance D on the one hand and the blood flow rate Qb and dialysate rate Qd on the other hand is described by the following equation:

$$K = Q_b Q_d \frac{1 - \exp\left(-k0A \frac{Q_d - Q_b}{Q_d Q_b}\right)}{Q_d - Q_b \exp\left(-k0A \frac{Q_d - Q_b}{Q_d Q_b}\right)}$$

where k0A is a coefficient.

24. Method according to claim 23, **characterised in that** different values of the coefficient k0A are read out from a memory unit (26) for different types of dialysers.

25. Method according to claim 24, **characterised in that**, for a preset blood flow rate Qb and a preset dialysate rate Qd, the clearance K or dialysance D is measured and the coefficient k0A is calculated.

26. Computer program product for a control device of an extracorporeal blood treatment device, **characterised in that** the method steps according to any of claims 18 to 25 are carried out when the control device is operated using the program.

## Revendications

1. Dispositif de commande d'un système de traitement sanguin extracorporel,
   dans lequel le système de traitement sanguin présente :

   une unité d'échange qui est subdivisée par une membrane semi-perméable en une première chambre et une deuxième chambre, dans lequel la première chambre fait partie d'une circulation sanguine extracorporelle dans laquelle une pompe de sang est disposée pour l'acheminement du sang à un débit de sang Qb déterminé,
   et/ou des moyens pour l'acheminement d'un liquide de dialyse à un débit de liquide de dialyse Qd déterminé à travers la deuxième chambre de l'unité d'échange,
   et/ou des moyens pour l'acheminement d'un substitut à un débit de substitut Qs déterminé dans la circulation sanguine extracorporelle,
   et/ou des moyens de retrait de l'ultrafiltrat de la première chambre au travers de la membrane semi-perméable dans la deuxième chambre de l'unité d'échange à un débit de liquide qui correspond à la somme du débit de substitut Qs et d'un débit d'ultrafiltrat Qf déterminé,

   dans lequel le dispositif de commande du système de traitement sanguin présente :

   une unité de commande (13) pour générer un signal de commande pour le réglage du débit d'acheminement de la pompe de sang à un débit de sang Qb déterminé et pour générer un signal de commande pour le réglage d'au moins un débit déterminé dans le groupe de débits parmi un débit de liquide de dialyse Qd, un débit d'ultrafiltrat Qf et un débit de substitut Qs,
   une unité de mémoire (26) pour mémoriser une clairance K ou une dialysance D prédéterminée,
   caractérisé en ce
   qu'une unité de calcul (13') est prévue, qui est conçue de telle sorte
   que pour au moins un débit prédéterminé du groupe des débits parmi un débit de sang Qb un débit de liquide de dialyse Qd, un débit d'ultrafiltrat Qf et un débit de substitut Qs, on calcule au moins l'un des autres débits respectifs du groupe des débits parmi un débit de sang Qb, un débit de liquide de dialyse Qd, un débit d'ultrafiltrat Qf et un débit de substitut Qs, uniquement sur la base d'une dépendance prédéterminée de la clairance K ou de la dialysance D aux débits pour lesquels la clairance K ou la dialysance D prédéterminée est conservée.

2. Dispositif selon la revendication 1, **caractérisé en ce que** l'unité de calcul (13') calcule en continu l'au moins un des autres débits respectifs du groupe des débits parmi un débit de sang Qb, un débit de liquide de dialyse Qd, un débit d'ultrafiltrat Qf et un débit de substitut Qs lors d'une modification de l'au moins un débit prédéterminé par l'unité de commande (13) au moins pour une periode de temps du traitement sanguin.

3. Dispositif selon la revendication 1, **caractérisé en ce que** l'unité de commande (13) coopère avec l'unité de calcul (13') de telle sorte que l'unité de commande (13) génère des signaux de commande pour le réglage de l'au moins un débit calculé dans le groupe des débits parmi un débit de sang Qb, un débit de liquide de dialyse Qd, un débit d'ultrafiltrat Qf et un débit de substitut Qs de sorte que l'au moins un débit soit réglé.

4. Dispositif de commande d'un système d'hémodialyse, dans lequel le système d'hémodialyse présente :

   un dialyseur qui est subdivisé par une membrane semi-perméable en une chambre de sang et une chambre de liquide de dialyse, et
   une pompe de sang pour l'acheminement du sang à travers la chambre de sang à un débit de sang déterminé et une pompe de liquide de dialyse pour l'acheminement du liquide de dialyse à travers la chambre de liquide de dialyse à un débit de liquide de dialyse déterminé,

   dans lequel le dispositif de commande du système d'hémodialyse présente :

   une unité de commande (13) pour générer un signal de commande pour le réglage du débit d'acheminement de la pompe de sang à un débit de sang Qb déterminé et pour générer un signal de commande pour le réglage du débit d'acheminement de la pompe de liquide de dialyse à un débit de liquide de dialyse Qd déterminé, et
   une unité de mémoire (26) pour mémoriser une clairance K ou une dialysance D prédéterminée,
   caractérisé en ce
   qu'une unité de calcul (13') est prévue, qui est conçue de telle sorte
   que, pour un débit de sang Qb déterminé, le débit de liquide de dialyse Qd soit déterminé uniquement sur la base d'une dépendance prédéterminée de la clairance ou de la dialysance au débit de sang et au débit de liquide de dialyse, pour lequel avec le débit de sang prédéterminé, une clairance K ou une dialysance D prédéterminée est conservée, et/ou
   que pour un débit de liquide de dialyse Qd prédéterminé, le débit de sang Qb soit déterminé uniquement sur la base d'une dépendance prédéterminée de la clairance ou de la dialysance au débit de sang et au débit de liquide de dialyse, pour lequel, avec le débit de liquide de dialyse prédéterminé, une clairance K ou une dialysance D prédéterminée est conservée.

5. Dispositif selon la revendication 4, **caractérisé en ce que** l'unité de calcul (13') calcule en continu le débit de sang Qb ou le débit de liquide de dialyse Qd lors d'une modification du débit de liquide de dialyse Qd ou du débit de sang Qb prédéterminé par l'unité de commande au moins pour une période de temps du traitement sanguin.

6. Dispositif selon l'une des revendications 1 à 5, caractérisé en ce
   que l'unité de commande (13) génère des signaux de commande pour actionner la pompe de liquide de dialyse à un débit d'acheminement de telle sorte que le débit de liquide de dialyse Qd déterminé soit réglé, et/ou
   l'unité de commande (13) génère des signaux de commande pour actionner la pompe de sang à un débit d'acheminement de telle sorte que le débit de sang Qb déterminé soit réglé.

7. Dispositif selon l'une des revendications 1 à 6, **caractérisé en ce qu'**une unité d'entrée (24) pour l'entrée d'une clairance K ou d'une dialysance D souhaitée coopère avec l'unité de mémoire (26) de telle sorte que la clairance ou la dialysance entrée soit mémorisée dans l'unité de mémoire, dans lequel l'unité de commande (13) prend en compte la clairance ou la dialysance entrée en tant que clairance ou dialysance prédéterminée.

8. Dispositif selon l'une des revendications 1 à 7, **caractérisé en ce qu'**une unité d'entrée (24) pour l'entrée d'un débit de sang et/ou d'un débit de liquide de dialyse souhaité coopère avec l'unité de commande (13) de telle sorte que des signaux de commande pour l'actionnement de la pompe de sang et/ou de la pompe de liquide de dialyse au débit de sang Qb et/ou au débit de liquide de dialyse Qd entrés soient générés.

**9.** Dispositif selon l'une des revendications 1 à 8, **caractérisé en ce que** l'unité de calcul (13') est conçue de telle sorte que lors d'un accroissement du débit de sang Qb le débit de liquide de dialyse Qd soit réduit et lors d'une réduction du débit de sang Qb le débit de liquide de dialyse Qd soit accru de telle sorte que la clairance K ou la dialysance D prédéterminée soit conservée, et/ou **en ce que** lors d'un accroissement du débit de liquide de dialyse Qd le débit de sang Qb soit réduit et lors d'une réduction du débit de liquide de dialyse Qd le débit de sang Qb soit accru de telle sorte que la clairance ou la dialysance prédéterminée soit conservée.

**10.** Dispositif selon l'une des revendications 4 à 9, **caractérisé en ce que** le rapport entre la clairance K ou la dialysance D prédéterminée d'une part et le débit de sang Qb et le débit du liquide de dialyse Qd d'autre part est décrit avec l'équation suivante :

$$K = Q_b Q_d \frac{1 - \exp\left(-k0A\dfrac{Q_d - Q_b}{Q_d Q_b}\right)}{Q_d - Q_b \exp\left(-k0A\dfrac{Q_d - Q_b}{Q_d Q_b}\right)}$$

dans laquelle KOA est un coefficient.

**11.** Dispositif selon la revendication 10, **caractérisé en ce que**, dans l'unité de mémoire (26), pour différents types de dialyseurs, différentes valeurs sont mémorisées pour le coefficient k0A, lesquelles sont prises en compte par l'unité de commande (13).

**12.** Dispositif selon la revendication 11, **caractérisé en ce que** l'unité de commande (13) coopère avec une unité de mesure (21) de telle sorte que pour un débit de sang Qb et un débit de liquide de dialyse Qd prédéterminés par l'unité de commande avec la clairance K ou la dialysance D déterminée par l'unité de mesure le coefficient k0A soit calculé.

**13.** Système de traitement sanguin comportant
une unité d'échange qui est subdivisée par une membrane semi-perméable en une première chambre et une deuxième chambre, dans lequel la première chambre fait partie d'une circulation sanguine extracorporelle dans laquelle une pompe de sang est disposée pour l'acheminement du sang à un débit de sang Qb déterminé,
et/ou des moyens pour l'acheminement d'un liquide de dialyse à un débit de liquide de dialyse Qd déterminé à travers la deuxième chambre de l'unité d'échange,
et/ou des moyens pour l'acheminement d'un substitut à un débit de substitut Qs déterminé dans la circulation sanguine extracorporelle,
et/ou des moyens de retrait de l'ultrafiltrat de la première chambre au travers de la membrane semi-perméable dans la deuxième chambre de l'unité d'échange à un débit de liquide qui correspond à la somme du débit de substitut Qs et d'un débit d'ultrafiltrat Qf déterminé,
**caractérisé en ce que** le système de traitement sanguin présente une unité de commande selon l'une des revendications 1 à 3.

**14.** Système d'hémodialyse comportant
un dialyseur (1) qui est subdivisé par une membrane semi-perméable (2) en une chambre de sang (3) et une chambre de liquide de dialyse (4),
une pompe de sang (6) pour l'acheminement du sang à travers la chambre de sang (3) à un débit de sang Qb déterminé et une pompe de liquide de dialyse (12) pour l'acheminement du liquide de dialyse à travers la chambre de liquide de dialyse (4) à un débit de liquide de dialyse Qd déterminé,
**caractérisé en ce que** le système d'hémodialyse présente une unité de commande selon l'une des revendications 4 à 12.

**15.** Procédé de commande d'un système de traitement sanguin extracorporel, dans lequel le système de traitement sanguin présente :

une unité d'échange qui est subdivisée par une membrane semi-perméable en une première chambre et une deuxième chambre, dans lequel la première chambre fait partie d'une circulation sanguine extracorporelle dans laquelle une pompe de sang est disposée pour l'acheminement du sang à un débit de sang Qb déterminé, et/ou des moyens pour l'acheminement d'un liquide de dialyse à un débit de liquide de dialyse Qd déterminé à travers la deuxième chambre de l'unité d'échange, et/ou des moyens pour l'acheminement d'un substitut à un débit de substitut Qs déterminé dans la circulation sanguine extracorporelle, et/ou des moyens de retrait de l'ultrafiltrat de la première chambre au travers de la membrane semi-perméable dans la deuxième chambre de l'unité d'échange à un débit de liquide qui correspond à la somme du débit de substitut Qs et d'un débit d'ultrafiltrat Qf déterminé,

comportant les étapes de procédé suivantes :

mémorisation d'une clairance K ou d'une dialysance D prédéterminée, calcul pour un débit prédéterminé du groupe des débits parmi un débit de sang Qb, un débit de liquide de dialyse Qd, un débit d'ultrafiltrat Qf et un débit de substitut Qs, d'au moins l'un des autres débits respectifs du groupe de débits parmi un débit de sang Qb, un débit de liquide de dialyse Qd, un débit d'ultrafiltrat Qf et un débit de substitut Qs, uniquement sur la base d'une dépendance prédéterminée de la clairance K ou de la dialysance D aux débits pour lesquels la clairance K ou la dialysance D prédéterminée est conservée.

16. Procédé selon la revendication 15, **caractérisé en ce que** l'au moins un des autres débits respectifs du groupe des débits parmi un débit de sang Qb, un débit de liquide de dialyse Qd, un débit d'ultrafiltrat Qf et un débit de substitut Qs est calculé en continu lors d'une modification de l'au moins un débit prédéterminé, au moins pour une période de temps du traitement sanguin.

17. Procédé selon la revendication 16, **caractérisé en ce que** l'au moins un débit calculé dans le groupe des débits parmi un débit de sang Qb, un débit de liquide de dialyse Qd, un débit d'ultrafiltrat Qf et un débit de substitut Qs est réglé.

18. Procédé de commande d'un système d'hémodialyse, dans lequel le système d'hémodialyse présente :

un dialyseur qui est subdivisé par une membrane semi-perméable en une chambre de sang et une chambre de liquide de dialyse, et une pompe de sang pour l'acheminement du sang à travers la chambre de sang à un débit de sang Qb déterminé et une pompe de liquide de dialyse pour l'acheminement de liquide de dialyse à travers la chambre de liquide de dialyse à un débit de liquide de dialyse Qd déterminé,

comportant les étapes suivantes :

mémorisation d'une clairance K ou d'une dialysance D prédéterminée, détermination, pour un débit de sang Qb prédéterminé, uniquement sur la base d'une dépendance prédéterminée de la clairance ou de la dialysance au débit de sang et au débit de liquide de dialyse, du débit de liquide de dialyse Qd pour lequel, avec le débit de sang prédéterminé, une clairance K ou une dialysance D prédéterminée est conservée, et/ou détermination, pour un débit de liquide de dialyse Qd prédéterminé, uniquement sur la base d'une dépendance prédéterminée de la clairance ou de la dialysance au débit de sang et au débit de liquide de dialyse, du débit de sang Qb pour lequel, avec le débit de liquide de dialyse prédéterminé, une clairance K ou une dialysance D prédéterminée est conservée.

19. Procédé selon la revendication 18, **caractérisé en ce que** le débit de sang Qb ou le débit de liquide de dialyse Qd est calculé en continu lors d'une modification du débit de liquide de dialyse Qd ou du débit de sang Qb prédéterminé, au moins pour une période de temps du traitement sanguin.

20. Procédé selon la revendication 18 ou 19, **caractérisé en ce que** le débit de liquide de dialyse Qd ou le débit de sang Qb déterminé est réglé.

21. Procédé selon l'une des revendications 18 à 20, **caractérisé en ce qu'**une clairance K ou une dialysance D souhaitée est entrée, dans lequel la clairance ou la dialysance entrée est la clairance ou la dialysance prédéterminée.

22. Procédé selon l'une des revendications 18 à 21, **caractérisé en ce que**, lors d'un accroissement du débit de sang Qb, le débit de liquide de dialyse Qd est réduit et, lors d'une réduction du débit de sang, le débit de liquide de dialyse est accru de telle sorte que la clairance K ou la dialysance D prédéterminée soit conservée et/ou **en ce que**, lors d'un accroissement du débit de liquide de dialyse Qd, le débit de sang Qb est réduit et, lors d'une réduction du débit de liquide de dialyse, le débit de sang est accru de telle sorte que la clairance ou la dialysance prédéterminée soit conservée.

23. Procédé selon la revendication 22, **caractérisé en ce que** le rapport entre la clairance K ou la dialysance D prédéterminée d'une part et le débit de sang Qb et le débit de liquide de dialyse Qd d'autre part est décrit avec l'équation suivante :

$$K = Q_b Q_d \frac{1 - \exp\left(-k0A\dfrac{Q_d - Q_b}{Q_d Q_b}\right)}{Q_d - Q_b \exp\left(-k0A\dfrac{Q_d - Q_b}{Q_d Q_b}\right)}$$

dans laquelle K0A est un coefficient.

24. Procédé selon la revendication 23, **caractérisé en ce que**, pour différents types de dialyseurs, différentes valeurs pour le coefficient k0A sont lues à partir d'une unité de mémoire.

25. Procédé selon la revendication 24, **caractérisé en ce que**, pour un débit de sang Qb et un débit de liquide de dialyse Qd prédéterminés, la clairance K ou la dialysance D est mesurée et le coefficient k0A est calculé.

26. Produit de programme informatique pour un dispositif de commande d'un système de traitement sanguin extracorporel, **caractérisé en ce que** les étapes de procédé selon l'une des revendications 18 à 25 sont réalisées lorsque le dispositif de commande est actionné avec le programme.

**Fig. 1**

**Fig. 2a**

**Fig. 2b**

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0428927 A1
- US 6702774 B1
- DE 19739100 C1
- US 5092836 A
- WO 2004022135 A1
- US 5744031 A
- US 20030230533 A1
- US 20030230533 A

**In der Beschreibung aufgeführte Nicht-Patentliteratur**

- Blutreinigungsverfahren. Georg-Thieme-Verlag, 1990, 11-13
- **SIGDELL J. ; TERSTEEGEN, B.** Clearance of a Dialyzer under varying Operation Conditions. *Artificial Organs,* 1986, vol. 10 (3), 219-225
- **WERYNSKI A. ; WANIEWSKI, J.** Theoretical Description of Mass Transport in Medical Membrane Devices. *Artificial Organs,* 1995, vol. 19, 420-427